(19) Europäisches Patentamt

European Patent Office

Office européen des brevets

(11) **EP 4 728 962 A1**

(12) **EUROPEAN PATENT APPLICATION**

(43) Date of publication:
**22.04.2026 Bulletin 2026/17**

(21) Application number: **24207530.7**

(22) Date of filing: **18.10.2024**

(51) International Patent Classification (IPC):
**A61B 1/005** (2006.01)  **A61B 34/20** (2016.01)
**G01B 11/24** (2006.01)

(52) Cooperative Patent Classification (CPC):
**A61B 34/20; A61B 1/009; G01B 11/24;**
A61B 2034/2061

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
 • **VAN DEN ENDE, Daan Anton**
 **Eindhoven (NL)**

 • **CLABBERS, Tom Johannes Hubertus**
 **Eindhoven (NL)**
 • **MARCELIS, Bout**
 **5656AG Eindhoven (NL)**
 • **DIJKKAMP, Dirk**
 **Eindhoven (NL)**
 • **HENDERSON, Eric Brett**
 **Eindhoven (NL)**

(74) Representative: **Philips Intellectual Property & Standards**
 **High Tech Campus 52**
 **5656 AG Eindhoven (NL)**

(54) **ELONGATED DEVICE FOR MEDICAL INTERVENTIONAL APPLICATION**

(57) The present invention relates to an elongated device (10) for medical interventional application. The device comprises a multicore optical fiber (12) arranged for sensing shape, a longitudinal lumen (14) having a longitudinal axis and embedding the optical fiber (12), a shell (16) surrounding the lumen (14), the shell (16) having a main section (18) and a distal terminal section (20) having a bending stiffness lower than that of the main section. The device (10) further comprises a mechani- cally bendable extension member (34) coupled to a distal ending portion (32) of the optical fiber (12), the extension member (34) longitudinally extending within the lumen (14) into the terminal section (20) of the shell (16). The extension member (34) is configured to transfer bending information related to a bending of the extension member (34) to the ending portion (32) of the optical fiber (12) such that the bending information is sensed by the optical fiber (12).

FIG.1

EP 4 728 962 A1

**Description**

FIELD OF THE INVENTION

The present invention generally relates to the field of optical shape sensing technology, also referred to as Fiber Optic RealShape (FORS) technology. In particular, the present invention relates to an elongated device for medical interventional application which is FORS enabled.

BACKGROUND OF THE INVENTION

Fiber Optic RealShape (FORS) technology is a shape sensing and reconstruction technology used in elongated devices, like guidewires, to enable accurate, three-dimensional and real-time visualization of the devices to allow efficient navigation during minimally invasive endovascular procedures without the need for X-ray guidance. The reduction of X-ray radiation exposure during such a procedure is beneficial for both patients as well as physicians and staff.

The FORS technology uses a shape sensing enabled elongated device, like a guidewire, for use in the procedure and a measurement system, which typically comprises a light source and an optical detector compatible with the shape sensing enabled elongated device, as well as a signal processing unit and a user interface for viewing and tracking the visualized elongated device.

FORS-enabled devices typically include an integrated twisted multicore optical glass fiber with sensors which may be configured as distributed fiber Bragg grating (FBG) sensors. Light from the light source is used to interrogate the sensors and reflections are analyzed to determine the local deformation of each segment of the optical fiber such that the full three-dimensional shape can be reconstructed. In this way, the full shape of elongated devices can be reconstructed and visualized in real time during the procedure. The FORS enabled devices may be visualized in the context of the patient's anatomy through overlay on images acquired before or during the procedure, like CT scans and X-rays. The amount of images are thus significantly reduced compared to X-ray guided procedures where a new X-ray image is needed every time the elongated device needs to be visualized, leading to a marked reduction in X-ray dose.

The applicant of the present application has developed FORS enabled guidewires and catheters, which have been successfully employed in complex aortic interventional procedures, in particular fenestrated endovascular aortic aneurysm repair (FEVAR) and/or branched endovascular aortic aneurysm repair (BEVAR), as well as in endovascular peripheral lesion repair (EVPLR).

In complex aortic procedures the requirements for mechanical properties of the tip section of the typically used guidewires do not restrict the use of brittle glass fiber sensors which extend up to the distal tip of the elongated device, since navigation often is within large vessels (e.g. aorta), where the guidewire is free to navigate inside the vessel and will typically not encounter obstructions.

In other procedures, such as coronary or various peripheral procedures, such as treatment of peripheral artery disease (PAD), FORS enabled elongated devices are also beneficial for reducing radiation dose. However, in these types of procedures, vessels are much smaller in diameter, and often the trajectory of navigation is very tortuous, i.e. with many bends, and potentially many bifurcations must be accessed to get to the target location, often involving acute angles through which the elongated device must travel. Moreover, these smaller vessels are much more fragile and may be damaged by the tip of the elongated device. Especially in coronary diseases and PAD, angioplasty techniques require crossing a narrowed or occluded section of a blood vessel. For more severe occlusions, crossing a narrowed or occluded section of a blood vessel requires force, and sometimes the tip of the device prolapses, i.e. the device loops and the tip of the device bends back on itself, a phenomenon also called 'knuckling', causing very tight bending in the device tip, i.e. a bending radius < 1 mm. In these cases, the device tip must also be robust enough to withstand this deformation.

For FORS enabled devices, like guidewires, used in peripheral interventional procedures, the tip of the device must be soft and flexible enough to easily navigate through tight bends and acutely angled bifurcations. For example, the tip load of the combination of the inner optical fiber and the outer shell tip of the device must not exceed the limits required for safe navigation, i.e. without damaging the blood vessels, which is around 2-4 gram force for guidewires used in peripheral procedures.

Also during these procedures, the curvature of the optical fiber may become high enough to impede shape sensing or may even become higher than the maximum curvature that the glass fiber can withstand, resulting in failure of the glass material, i.e. breaking or shattering, during use.

In particular, during procedures where a tortuous anatomy must be navigated and/or a narrow lesion or occlusion must be crossed the forces acting on the tip of the device may result in deformations of the device tip which are too extreme for a glass fiber to handle, especially when the device tip is looped or prolapsed. Breaking of the glass fiber will not only impede the use of the FORS technology, but may also result in a patient safety issue if the glass splinters potentially damage the internal structure of the device tip, further altering its mechanical properties.

At the same time, the shape and direction of the tip of the elongated device must still be reconstructed by the FORS technology as accurate as possible even under such challenging circumstances, involving large deformations due to looping and prolapse, resulting in a tight bending radius, such that the need for X-ray guidance can still be avoided,.

SUMMARY OF THE INVENTION

It is an object of the present invention to provide an elongated device for medical interventional application, suitable for procedures where a tortious anatomy must be navigated and/or a narrow lesion or occlusion must be crossed, while at the same time allowing reconstruction of the shape and direction of the tip region of the elongated device by the FORS technology as accurate as possible.

In an aspect of the present invention, an elongated device for medical interventional application is provided, comprising:

a main section and a distal terminal section having a bending stiffness lower than that of the main section,
an optical fiber arranged for sensing shape and arranged in the main section,
a bendable extension member coupled to a distal ending portion of the optical fiber, the extension member longitudinally extending within the terminal section, the extension member being configured to transfer bending information related to a bending of the extension member to the distal ending portion of the optical fiber such that the bending information is sensed by the optical fiber. The bendable extension member may be, in one embodiment, a mechanically bendable extension member or a bendable mechanical extension member.

The present invention proposes a new technical design of an elongated device for medical interventional application. The elongated device according to the invention is particularly suitable for coronary and peripheral procedures where the elongated device has to be navigated through tight bends and acutely angled bifurcations. The optical fiber of the elongated device according to the invention does not extend up to the distal tip of the elongated device, but its distal end terminates spaced apart from the distal tip of the elongated device. In order to provide for a shape sensing capability of the elongated device up to the distal tip of the elongated device, the elongated device according to the invention comprises a (mechanical) extension member coupled to the optical fiber at the distal ending portion of the optical fiber. The extension member may be a purely mechanical element, i.e. it does not need to be shape sensing enabled for itself, but only needs to be bendable and suited to couple a deformation of the terminal section of the device into the optical fiber. The extension member may have a bending stiffness, also known as flexural rigidity, which is lower than the bending stiffness of the optical fiber. The terms 'bending stiffness' and 'flexural rigidity' are used synonymously in the present disclosure. Preferably, the extension member may be much more flexible than the optical fiber. However, the extension member is stiff enough to transfer bending information from the extension member to the ending portion of the optical fiber, i.e. it is stiff enough that a deformation of the extension member induces strain in the ending portion of the optical fiber which is sensed by the optical fiber. In other words, when the distal terminal section of the device and, thus, the extension member deforms, the deformation of the extension member is conveyed as bending information to the distal ending portion of the optical fiber, and the bending information conveyed is sensed by the optical fiber as strain in the optical fiber. The bending information can be a bending direction and/or a magnitude of the bending. The extension member may also be more resistant to fracture (breaking) than the optical fiber, and in that case, the extension member may principally have the same or even a higher bending stiffness than the optical fiber.

In the present disclosure, bending deformation is described as 'bending radius' as well as 'bending curvature'. Both terms relate to bending of the optical fiber or device, and the relation between both terms is defined as: low (or tight) bending radius equals high curvature and vice versa.

The bending information from the extension member and, thus the distal terminal section of the elongated device, may be identified by a data processor that measures and/or at least estimates the bending of the terminal section of the device from the identified bending information.

Preferably, the bendable extension member is flexible and robust enough that it can loop and prolapse as described above without fracturing.

Further, the elongated device according to the invention comprises a main section which preferentially protects the optical fiber, and a soft distal terminal section which has a bending stiffness (flexural rigidity) lower than that of the main section. Thus, the distal terminal section into which the bendable extension member extends, can loop and prolapse so that the distal tip of the elongated device can cross a narrowed or occluded section of a blood vessel which may cause a very tight bending in the tip section of the elongated device without damaging the blood vessel.

Preferred embodiments of the invention are defined in the dependent claims and/or are described in the following.

Preferably, the extension member may be a high strength stainless steel or nitinol elongated element. The extension member may be a wire.

In an embodiment, the elongated device may be a guidewire.

In a further embodiment, the extension member may have a length in a range from 1 cm to 5 cm.

In a further embodiment, the extension member may extend from the ending portion of the optical fiber up to a distal end of the terminal section of the elongated device.

It is advantageous here that bending information can be conveyed by the extension member from the tip of the terminal section of the device to the optical fiber so that bending information related to a deflection of the distal end of the device can be conveyed to the optical fiber. Thus, shape and direction (orientation) of the tip of the elongated device can be reconstructed by the FORS technology.

In a further embodiment, the extension member may have a cross-section smaller than the cross-section of the optical fiber.

A thin extension member with a small diameter is advantageous because of an increased flexibility of the extension member so that the extension member is able to loop, e.g. in a procedure of crossing an occlusion or tight bending of the vessel.

In a further embodiment, an inner wall of at least one of the main section and the terminal section may have one or more protrusions at pre-determined positions along the at least one of the main section and terminal section to limit movement of at least one of the extension member and the optical fiber in one or more directions transverse to a longitudinal axis of the extension member or optical fiber.

This measure has the advantage that the accuracy of the estimation or measurement of the bending of the distal end of the elongated device is increased. A limitation of the free movement of the extension member in the terminal section provides for a more direct transfer of a deformation of the terminal section via the extension member to the ending portion of the optical fiber. In an advantageous example of this embodiment a protrusion may be provided at the distal end of the main section such that a lever mechanism is created at this position for controlling the deformation of the distal end of the optical fiber. Deformation induced in the optical fiber due to the bending of the extension member can be advantageously controlled in a more defined manner. Since the optical fiber is highly sensitive to deformation but does not have a lot of room to deflect within the main section of the device, it is advantageous to reduce the induced deformation in the optical fiber compared to the deformation of the extension member. It is also advantageous to provide a protrusion slightly proximally from the distal ending portion of the optical fiber which at that point restricts movement of the optical fiber so that only the more distally located ending portion of the optical fiber will experience bending deformation due to a deformation of the distal terminal section of the device.

In a further embodiment, a distal end of the terminal section may be configured to restrict movement of the distal end of the extension member in one or more directions transverse to a longitudinal axis of the extension member.

In this way, a transverse play or backlash of the extension member can be at least reduced so that a deformation of the device tip is advantageously directly transferred to the optical fiber

In a further embodiment, the extension member may be coupled to the optical fiber eccentrically with respect to a longitudinal center axis of the optical fiber.

This embodiment is advantageous, as a bending deformation of the extension member will induce a compressive or tensile strain in the optical fiber dependent on the direction of bending so that this embodiment allows for more accurately estimating the direction and magnitude of a deflection of the device tip.

In the context of the previous embodiment, it is further preferred if a distal end of the extension member is connected, preferably elastically connected, in particular attached, to a distal end of the shell eccentrically with respect to a longitudinal center axis of the lumen.

In this embodiment, bending information transfer from the distal tip of the device is further improved, because the distal end of the extension member cannot freely move in the terminal section of the device.

In the context of one or both of the previous embodiments, it is further preferred if the elongated device comprises a ribbon arranged along an inner wall of the terminal section of the device and having a first bending resistance in two opposite first bending directions and a second bending resistance in two opposite second bending directions transverse to the first bending directions, the first bending resistance being smaller than the second bending resistance, and wherein the extension member is arranged with respect to the ribbon such that the first bending directions are the preferential bending directions.

In this embodiment, the ribbon, occasionally also referred to herein as stripe-shaped element, provides a preferential direction of bending of the terminal section of the device and the extension member. For example, the ribbon may have a width dimension that is larger than the thickness dimension of the stripe-shaped element. The bending resistance in the width direction thus is higher than in the thickness direction. The different bending resistances in two orthogonal bending directions provides that there is only a small chance of the distal terminal section of the device being accidentally bent in the wrong direction, i.e. perpendicular to the eccentricity of the extension element. Thus, in a practical embodiment, the extension member is arranged with respect to the ribbon such that the extension member faces the wide side of the ribbon.

In a further embodiment, the extension member and/or a coupling element arranged between the distal ending portion of the optical fiber and the extension member may have an asymmetric cross-section such that bending of the extension member or coupling element induces twist in the ending portion of the optical fiber which twist is sensed by the optical fiber.

An optical fiber configured for shape sensing typically is sensitive to axial strain (compression/extension), bending strain, but also to twist (torsional) strain. If the extension member is configured such that it has an asymmetric cross-section, with the shear center off the symmetry axis, bending of the extension member induces a twist in the extension member which in turn is transferred to the optical fiber. The bending load on the extension member is related to the eccentricity of the shear center so that the twist inducing moment is equal to the bending load times the eccentricity. The twist can be sensed by the optical fiber, and from the sensed twist the bending of the extension member and thus of the distal terminal section of the shell can be at least estimated.

If such a bend-twist coupling cannot be provided by the extension member itself, for example if the extension member has a symmetric cross-section with no shear center eccentricity, an additional coupling element may be arranged between the distal ending portion of the optical fiber and the extension member, wherein the coupling element has an asymmetric cross-section such that bending of the extension member induces twist in the coupling element, wherein the twist in the additional coupling element is transferred to the ending portion of the optical fiber, where it is sensed.

In a further embodiment, the elongated device may comprise a plurality of extension members spatially distributed and coupled to the ending portion of the optical fiber.

This embodiment is advantageous in terms of an increased accuracy of the measurement of the bending of the terminal section of the device, as it allows for an improved spatial resolution of the measurement or estimation of the bending along the terminal section of the device. For example, if the terminal section bends in several segments thereof differently, for instance in the shape of an 'S' curve, it may be difficult to accurately predict the shape of the terminal section of the device. By combining a plurality of extension members spatially distributed and coupled to the ending portion of the optical fiber, the accuracy of the measurement or estimation of the shape of the distal terminal section can thus be improved.

In this context, further embodiments provide that the extension members of the plurality of extension members may be coupled to the ending portion of the optical fiber at different axial and/or circumferential positions along or around a longitudinal axis of the ending portion of the optical fiber, and/or the extension members of the plurality of extension members terminate at different axial and/or circumferential positions along or around a longitudinal axis of the terminal section of the device.

In this embodiment, the optical fiber can sense strain in each of these locations which strain in turn corresponds to a deformation of the terminal section at the corresponding connected position. An advantage of this embodiment is that several positions of the terminal section of the device may be sensed as to deformations in these positions so that, as an example, an 'S' shape of the terminal section can be measured or estimated. In this embodiment, the extension members may have equal or different lengths among each other.

In a further embodiment, the extension members of the plurality of extension members may be coupled to different cores of the distal ending portion of the optical fiber.

In this embodiment, each extension member will affect only one of the fiber cores of the optical fiber. If the extension elements differ in length, as provided in a further preferred embodiment, the spatial information of the bending deformation of the terminal section of the device is transferred to the optical fiber by the number of fiber cores that are strained. If the bending is farther away from the distal end of the optical fiber, less fiber cores will be strained. Although the resolution may be limited by cross-talking between the fiber cores, the advantage of this embodiment is a compact configuration of multiple measurement points.

In a further embodiment, the extension member may be configured to provide different strain responses to bending along a length of the extension member, wherein the extension member comprises at least one of a varying cross-section diameter along the length of the extension member, a varying cross-section shape along the length of the extension member, a varying symmetry or asymmetry of the cross-section of the extension member along the length of the extension member.

This embodiment is advantageous, as it only requires a single mechanical extension member, while spatial resolution of the measurement or estimation of the deformation of the terminal section of the device is nevertheless achieved. For example, an extension member which has different cross-sectional shapes in different sections along its length, provides that these sections of the extension member respond differently to bending and will exert different types of strain on the optical fiber. For instance, an extension member with three different cross-sections along its length may be connected to the optical fiber which can induce bending, extension/compression and twist strains into a sensor through different cross-sectional shapes.

It is to be understood that the embodiments described above can be combined with one another. For example, the bending direction of the extension member providing different strain responses to bending along its length may be aligned with a thin axis of a ribbon described above, in order to impart a preferred bending direction of the terminal section of the device.

The combined strains at the ending portion of the optical fiber, i.e. bending, axial and/or twist, can be related to both magnitude, direction and location of the bending deformation in the terminal section of the device further distal from the distal end of the optical fiber. Although this mechanical coupling by an extension member or extension members is not as accurate as direct strain sensing with the optical fiber, the device tip can easily prolapse since the extension member is much more robust than any glass fiber.

BRIEF DESCRIPTION OF THE DRAWINGS

These and other aspects of the invention will be apparent from and elucidated with reference to the embodiments described hereinafter. In the following drawings

Fig. 1 shows schematically an elongated device for medical interventional application in a longitudinal section in a distal region of the elongated device;

Fig. 2 shows schematically a further embodiment of an elongated device for medical interventional application in a longitudinal section in a distal region of the elongated device;

Fig. 3 shows schematically a further embodiment of an elongated device for medical interventional application in a longitudinal section in a distal region of the elongated device;

Figs. 4a) -d) schematically show embodiments of extension members providing a bend-twist coupling;

Fig. 5 shows schematically a further embodiment of an elongated device for medical interventional application in a longitudinal section in a distal region of the elongated device;

Fig. 6 shows schematically a further embodiment of an elongated device for medical interventional application with multiple extension members coupled to different cores of an optical fiber of the elongated device;

Fig. 7 shows schematically a further embodiment of an elongated device for medical interventional application in a longitudinal section in a distal region of the elongated device, wherein an outer shell of the elongated device is omitted;

Fig. 8 shows schematically a multicore fiber with 4 fiber cores;

Fig. 9a) -c) show diagrams illustrating the introduction of strain in an optical fiber by deformation of an extension member coupled to the optical fiber for different deformation states of the extension member; and

Fig. 10a) and b) show diagrams illustrating determination of shape of an extension member coupled to an optical fiber.

DETAILED DESCRIPTION OF THE INVENTION

In the following, multiple embodiments of shape sensing enabled elongated devices for medical interventional application will be described which are especially suitable for navigating tortuous vasculature and for crossing occlusions in a vessel.

In medical interventional applications, such as coronary or other peripheral procedures, such as treatment of peripheral artery disease, Fiber Optic RealShape (FORS) enabled elongated devices, especially FORS enabled guidewires, are beneficial for reducing radiation dose. However, in these types of procedures, vessels are very small in diameter, and often the trajectory of navigation is very tortuous (i.e. with many bends), and potentially many bifurcations must be accessed to get to the target location, often involving acute angles through which the elongated device must travel and, for example, a catheter subsequently must follow. Moreover, these smaller vessels are much more fragile and may be damaged by the tip of the elongated device. Especially in PAD, angioplasty techniques require crossing a narrowed or occluded section in a blood vessel. For more severe occlusions, this typically requires force and sometimes the tip of the wire prolapses, i.e. the wire loops and the tip bends back from itself, a phenomenon also called 'knuckling' which causes very tight bending in the tip of the device, e.g. a bending radius < 1 mm. The tip of the elongated device must also be robust enough to withstand this deformation.

Wang, Jian-Bo et al. "An Effective Guidewire Looping Technique for the Recanalization of Occlusive Segments of Infrapopliteal Vessels", Korean Journal of Radiology 11 (2010), pages 441-448, describe such a transluminal angioplasty technique. As described there, a hydrophilic guidewire is inserted into a proximal branch of the vessel and then a 'U' shape loop is made by rotating and advancing the guidewire continually. The looped guidewire is then advanced into and over occlusive segments of the vessel, whereafter a catheter is crossed over the occlusive segment along the guidewire. The guidewire described there is not equipped with an optical fiber.

When the elongated device, like a guidewire, includes an optical fiber extending up to the distal tip of the elongated device, and when the optic sensor is subject to a loop formation as described above, the curvature of the optical fiber may become too high so that shape sensing is impeded, or, when the curvature of the optical fiber becomes even higher than the maximum curvature that the glass fiber can withstand, this results in failure of the glass material of the optical fiber, i.e. the optical fiber may break or shatter.

The embodiments of the present invention, which will be described in the following, eliminate these drawbacks by providing a new structural design of an elongated device for medical interventional application. The elongated device according to the invention, especially a guidewire, comprises an embedded multicore optical fiber, which may have one or more fiber Bragg gratings, for sensing strain and deformation such that a 3D shape reconstruction of the optical fiber can be performed with an optical interrogator system. A mechanical extension member is located in the distal terminal section of the elongated device which extension member is connected to the optical fiber in such a way that a deformation of the extension member is coupled into the optical fiber so that bending deformation beyond the distal end of the optical fiber can be measured and/or estimated.

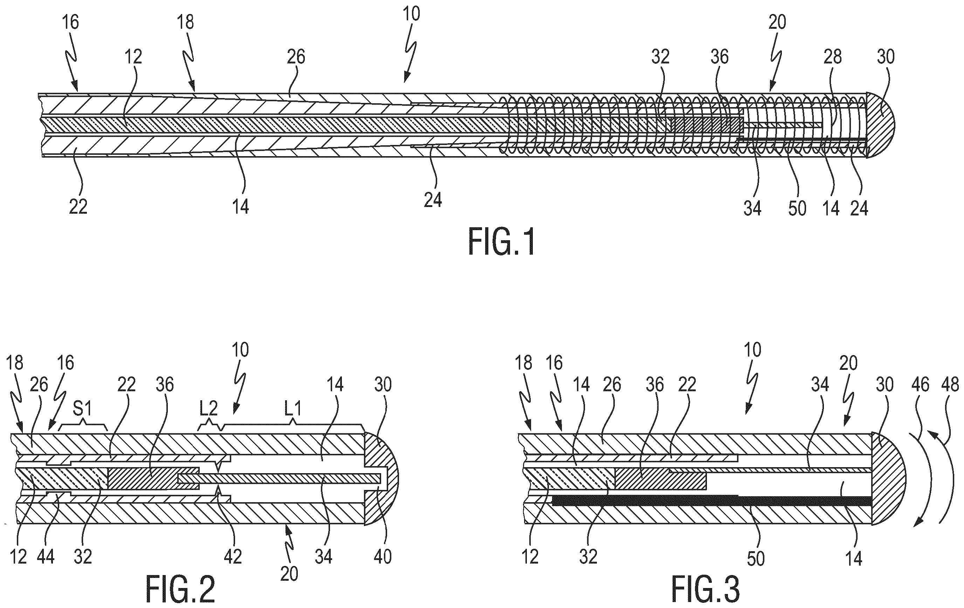

Fig. 1 shows a basic embodiment of an elongated device for medical interventional application which is labeled with general reference numeral 10. The elongated device 10 especially may be a guidewire. The device 10 is FORS enabled. Fig. 1 shows the elongated device 10 in a distal region thereof in an enlarged scale. The overall length of the elongated device may be more than 50 cm, more than 100 cm, more than 150 cm, and up to 300 cm or longer, for example. The diameter of the elongated device in its distal region may be smaller than 0.035", such as smaller than 0.025".

The elongated device 10 comprises an optical fiber 12 which is arranged for sensing shape of the optical fiber 12 and thus of the elongated device 10. The optical fiber 12 preferably is a multicore fiber, i.e. an optical fiber with a plurality, e.g. 4, fiber cores. The device 10 comprises a main section 18 and a distal terminal section 20 arranged distally from the main section 18. The optical fiber 12 is embedded in the main section 18 of the device 10. The main section 18 and/or the distal terminal section may comprise a lumen 14. The optical fiber 12 may be free to slide inside the lumen 14. The elongated device 10 may further comprise a shell 16 which surrounds the lumen 14. The terminal section 20 of the device 10 has a bending stiffness (flexural rigidity) which is lower than that of the main section 18. The shell 16 may comprise a tube 22, in particular a nitinol hypotube, which provides stiffness to the main section 18 of the device 10. The shell 16 may further comprise, in particular in the terminal section 20 of the device 10, a tube 24 made from a material which is more flexible than the tube 22. The tube 24 may be a polymer tube, which may be braided. A coating 26, for example a polyurethan (PU) coating may cover a part of the tube 22 and the tube 24. The elongated device 10 may further comprise a coil 28 embedded in the coating 26. An outermost distal end 30 of the elongated device may comprise a solder or glue, especially in a rounded, e.g. hemispherical, shape.

Differently from conventional FORS enabled elongated devices, the optical fiber 12 terminates in a distance apart from the outermost distal end 30 of the elongated device, i.e. a distal ending portion 32 of the optical fiber 12 is arranged spaced apart from the outermost distal end 30 of the elongated device 10. This means that the optical fiber 12 cannot directly measure the shape of the terminal section 20 of the elongated device 10 up to the distal end 30. However, an indirect measurement, or at least an estimation of the shape of the terminal section 20 of the elongated device 10 is made possible by a mechanical extension member 34 which is coupled to the distal ending portion 32 of the optical fiber 12. Coupling can include any type of direct or indirect connection of the extension member 34 with the distal ending portion 32 of the optical fiber 12 such that a deformation of the extension member 34 introduces strain in the optical fiber 12. Coupling of the extension member 34 to the distal ending portion 32 of the optical fiber 12 may be a direct coupling, or an indirect coupling via an additional coupling element between the extension member 34 and the distal ending portion 32 of the optical fiber 12. Direct coupling may be accomplished by glueing the extension member 34 to the distal ending portion 32 using an adhesive 36. The adhesive 36 may be an epoxy, for example.

The extension member 34 extends within the lumen 14 at least into the terminal section 20 of the device 10 and may extend up to the distal end 30 of the device 10 in some embodiments to be described later.

The extension member 34 may have a bending stiffness which is lower than the bending stiffness of the optical fiber 12, e.g. the extension member 34 may be much more flexible than the optical fiber 12. Nevertheless, the extension member 34 has enough stiffness to at least partly transfer bending information, which may be information about a direction and/or magnitude of bending of the terminal section 20, to the ending portion 32 of the optical fiber 12. The extension member 34 may be a high strength stainless steel or a nitinol wire.

The extension member 34 is coupled, e.g. attached to the fiber 12 such that the deformation of the terminal section 20 or deflection of the distal end 30, for instance when the tip coil 28 prolapses, is conveyed to the ending portion 32 of the optical fiber 12 to induce strain in the ending portion 32 of the optical fiber 12. The bending information sensed by the ending portion 32 of the optical fiber can be used, for example, by a signal processor (not shown) to estimate prolapsing of the terminal section 20 of the elongated device 10.

Preferably, the optical fiber 12 terminates (ending portion 32) before the terminal section 20 of the device 10. Also preferably, the optical fiber 12 terminates in the present embodiment close to a distal end 38 of the tube 22. The ending portion 32 of the optical fiber 12 may extend up to the distal end of the connection section 36 (epoxy).

The extension member 34 preferentially has a diameter smaller than a diameter of the optical fiber 12 to provide a sufficient flexibility of the extension member 34.

Further embodiments will be described with reference to Figs. 2 to 7. In the following description, elements which are identical, similar or comparable with elements in Fig. 1 are denoted with the same reference numerals. Some parts of the embodiment in Fig. 1 are not shown in Figs. 2-7, for example the coil 28, which however may also be present in the embodiments to be described hereinafter.

Fig. 2 shows an embodiment of an elongated device 10 which differs from the embodiment in Fig. 1 in that the extension member 34 extends up to the distal end 30 of the device 10. The distal end 30 of the terminal section 20 may be configured to restrict movement of the distal end of the extension member 34 in one or more directions transverse to a longitudinal axis of the extension member 34. For example, a cavity 40 may be provided in the distal end 30 of the elongated device 10 into which the extension member 34 extends as shown in Fig. 2. Thereby, the lateral movement of the extension member 34 in the lumen 14, i.e. transverse to the longitudinal axis of the lumen 14, is restricted so that a deflection of the distal end 30 of the elongated device 10 is directly transferred to the extension member 34 and via the extension member 34 to the optical fiber 12. In other words, the 'free travel' of the extension member 34 due to a lateral distance between the extension member 34 and the surface of the lumen 14 transverse to the longitudinal axis of the lumen 14 is reduced or even excluded.

Further, an inner wall of the terminal section 20, in the embodiment of Fig. 2 an inner wall of the tube 22, may have a protrusion 42 arranged in a proximal portion of the extension member 34 in vicinity of the distal end of the ending portion 32 of the optical fiber 12. The protrusion 42 projects inwardly to limit movement of the extension member 34 in one or more transverse directions, which results in a lever mechanism at the position of the protrusion 42. The lever mechanism controls the deformation of the ending portion 32 of the optical fiber 12.

A further protrusion 44 may be provided at the inner wall of the main section 18, here the inner wall of the tube 22, at a position of the ending portion 32 of the optical fiber 12. The protrusion 44 reduces free transverse movement of the optical fiber 12 in one or more directions transverse to the longitudinal axis of the lumen 14.

The protrusions 42 and 44 may extend around the extension member 34 and/or the ending portion 32 of the optical fiber 12 over a part of or over the full circumference of the lumen 14.

The protrusion 42 has the advantage that it controls the deformation induced in the optical fiber 12 due to a deformation of the terminal section 20 of the elongated device 10 by a ratio L2/L1. Since the FORS enabled optical fiber 12 typically is highly sensitive to deformation, but does not have a lot of room to deflect within the shell 16, here the tube 22, it is advantageous to reduce the induced deformation in the optical fiber 12 compared to the deformation of the distal end 30. The protrusion 44, which restricts movement of the optical fiber 12, has the advantage that only the more distally located section of the ending portion 32 of the optical fiber 12 will experience bending deformation due to a deformation of the distal end 30 of the device 10. The protrusion 44 restricts the strain in the optical fiber 12 due to bending to the location of S1 only.

While in the embodiments of Figs. 1 and 2 the extension member 34 is coupled to the ending portion 32 of the optical fiber 12 concentrically with respect to the optical fiber 12 and the lumen 14, Fig. 3 shows an embodiment of an elongated device 10, where the extension member 34 is coupled to the ending portion 32 of the optical fiber 12 eccentrically with respect to the optical fiber 12. The extension member 34 is also connected to the distal end 30 of the elongated device 12 eccentrically with respect to the longitudinal center axis of the lumen 14. A bending deformation of the terminal section 20 of the device 10 will induce an (extra) compressive or tensile strain in the optical fiber 12 dependent on the direction of bending. For example, bending of the terminal section 20 in direction of an arrow 46 will induce tensile strain in the optical fiber 12, while bending of the terminal section 20 in direction of an arrow 48 will induce compressive strain in the optical fiber 12. Preferably, the extension member 26 is connected, e.g. attached, in particular elastically connected, to the distal end 30 of the device 10.

The (extra) compressive/tensile strain induced in the ending portion 32 of the optical fiber 12 allows for estimating the direction and magnitude of the deflection of the distal end 30 of the device 10.

In the embodiment of Fig. 3, a stripe-shaped element or ribbon 50 may be arranged along an inner wall 52 of the shell 16 in the region of the terminal section 20 of the device 10. The ribbon 50 provides stability to the terminal section 20. The ribbon 50 has a first bending resistance in two opposite first bending directions (in Fig. 3 the two bending directions according to arrows 46 and 48), and a second bending resistance in two opposite second bending directions transverse to the first bending directions (which are bending directions transverse the plane of drawing in Fig. 3). The first bending resistance is smaller than the second bending resistance. The extension member 34 is arranged with respect to the ribbon 50 such that bending of the extension member 34 in the first bending directions (arrows 46 and 48) are preferential bending directions, while bending of the extension member 34 in the second bending directions is impeded. In this way, the ribbon 50 provides mechanical stability in the terminal section 20 of the device 10 in the second bending directions. The eccentric placement of the extension member 34 preferably aligns with the thin axis (thickness direction) of the ribbon 50. The difference in bending resistance of the thick axis and thin axis of the ribbon 50 will induce a preferential bending direction (here the bending direction according to arrows 46 and 48) of the distal end 30 of the device 10 such that the extension/compression of the optical fiber 12 coincides with the bending of the extension member 34 in the direction orthogonal to the width dimension of the ribbon 50. For instance, a ribbon with a width dimension that is 3 times larger than the thickness dimension, has a resistance to bending in the width direction which is 81 times higher than in the thickness direction so that there is only a small chance of the terminal section 20 (including the coil 28 in Fig. 1) to accidentally bend in the wrong direction, i.e. perpendicular to the eccentricity of the attached extension member 34.

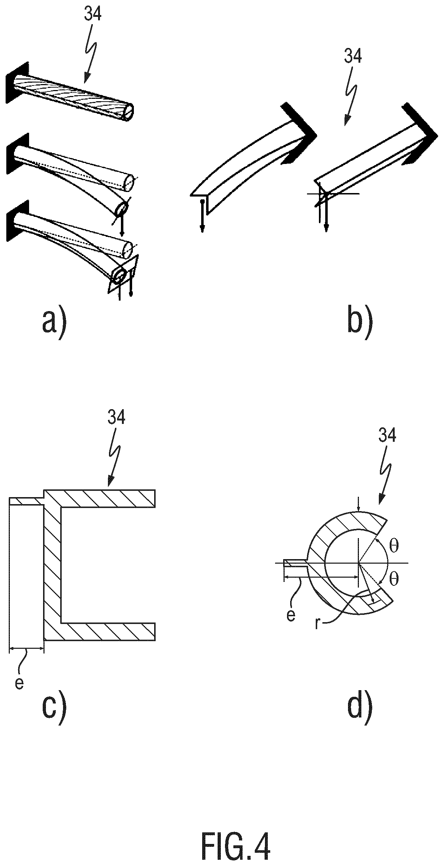

Instead of inducing pure bending strain or tensile/compressive strain in the ending portion 32 of the optical fiber 12, a bending deformation of the extension member 34 may also be coupled into the optical fiber 12 by a bend-twist coupling mechanism, inducing a rotation moment in the ending portion 32 of the optical fiber 12, as will be described with reference to Fig. 4a)-d).

A bend-twist coupling between the extension member 34 and the optical fiber 12 may be achieved by a configuration of the extension member 34 with an asymmetric cross-section, with a shear center eccentric to or off from its symmetry axis, such that bending of the extension member 34 induces twist in the extension member 34, which is transferred to the ending portion 32 of the optical fiber 12. The twist thus induced in the ending portion 32 can be sensed by the optical fiber 12. Fig. 4b) shows an extension member 34 having an L-shaped cross-section as an example for an eccentric shear center asymmetric cross-section. Fig. 4c) shows an example of an eccentric shear center asymmetric cross-section with eccentricity e configured as a rectangular open-channel profile. Fig. 4d) shows an example of an eccentric shear center asymmetric cross-section with eccentricity e configured as a circular open-channel profile. The eccentricity e is given by

$$e = 2r\ (cos\,\theta\ (2\pi - 2\theta)\ +\ 2sin\theta)/(2\pi - 2\theta + sin\ 2\theta) \hspace{2cm} (1)$$

Fig. 4a) shows an example of an extension member 34, in which the extension member 34 is made of a composite structure having an angled composite layup or asymmetric cross-section. The shear center is off from the symmetry axis.

The twist induced by a bending load into the extension member 34 is related to the eccentricity of the shear center such that the twist inducing moment is equal to the bending load times the eccentricity.

For instance, in the embodiment shown in Fig. 1, the extension member 34 may have an asymmetric cross-section with a shear center eccentric to the central axis of the optical fiber 12. The cross-section of the extension member 34 may be a circular open-channel like the one in Fig. 4d). When the terminal section 20 or distal end 30 of the device 10 deforms in bending, thereby deforming the extension member 34 in bending also, the asymmetry of the cross-section of the extension member 34 will induce a twist, resulting in a torsional moment and acting on the ending portion 32 of the optical fiber 12. For an extension member with length $L$ and bending stiffness $EI,$ the equivalent force F to achieve a certain deformation $d$ is $F = 3dEI/L^3$ (assuming only the distal end of the extension member 34 is loaded, as in the embodiment of Fig. 2, where the extension member 34 extends into the cavity 40 of the distal end 30 of the device 10). Then, with the eccentricity e given in equation (1) above, the torsional moment $M$ induced by the twist coupling is $M = Fe = 6dEIr\,(cos\,\theta\ (2\pi - 2\theta) + 2sin\theta)/L^3(2\pi - 2\theta + sin\ 2\theta)$. Thus, the optical fiber 12 at the ending portion 32 will sense a twisting moment $M$ as a function of deformation d such that the magnitude and direction of the bending may be estimated from the twist in the ending portion 32 of the optical fiber 12 induced by the twisting moment M acting on the distal end portion 32 of the optical fiber 12.

Instead of providing the extension member 34 as a bend-twist coupling member, it is also possible to provide an additional coupling element (not shown) arranged between the distal ending portion 32 of the optical fiber 12 and the extension member 34, wherein the coupling element has an asymmetric cross-section such that bending of the extension member 34 induces twist in the coupling element and thereby in the ending portion 32 of the optical fiber 12 which in turn can be sensed by the optical fiber 12.

While the embodiments described above provide a certain accuracy of the measurement or at least estimation of the bending of the terminal section 20 of the device 10, further embodiments will be described hereinafter which provide for an increased accuracy of the measurement or estimation in terms of spatial resolution. If the terminal section 20 of the device 10 bends in several segments along its length differently, for example like an 'S' curve, it is difficult to accurately predict the shape of the terminal section 20 as a whole.

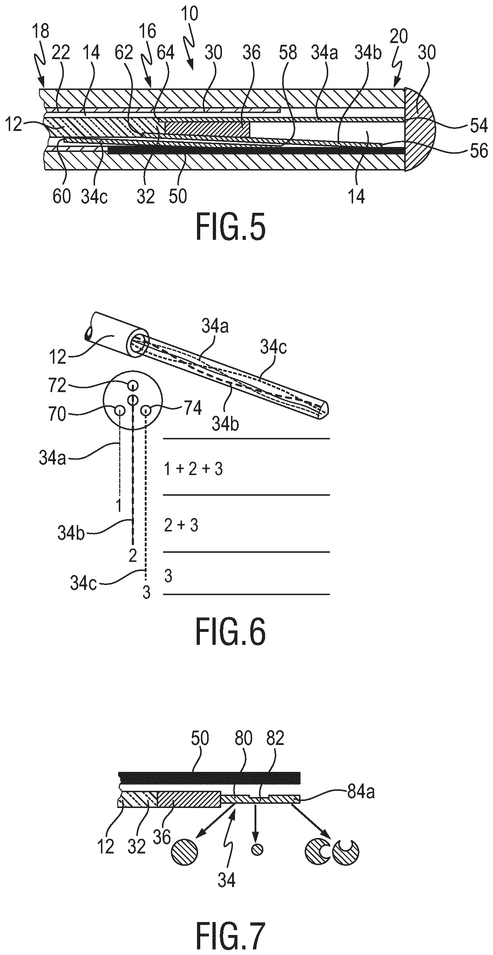

In order to increase accuracy of measuring or estimating a deformation of the terminal section 20 of the device 10, in particular when the terminal section 20 and thus the extension member 34 is designed to be longer, the embodiment of Fig. 5 provides multiple extension elements 34a, 34b, 34c that are combined to improve the spatial resolution of the bending information transferred from the terminal section 20 by the extension members 34a-c to the ending portion 32 of the optical fiber 12. Although three extension members 34a, 34b, 34c are shown, the number of extension elements may be two or more than three.

The extension members 34a-34c are coupled to the ending portion 32 of the optical fiber 12 at spatially distributed locations 60, 62, 64. The locations 60, 62, 64 may include different axial and/or circumferential positions along or around the longitudinal axis of the ending portion 32 of the optical fiber 12. The extension members 34a-34c may distally terminate at different axial and/or circumferential positions 54, 56, 58 along or around the longitudinal axis of the terminal section 20 of the device 10. Strain measured at the locations 60, 62, 64 of the ending portion 32 of the optical fiber 12 corresponds to deformation of the terminal section 20 at the corresponding connected locations 54, 56, 58. The advantage of this design is that a plurality of positions beyond the distal end portion 32 of the optical fiber may be sensed, thus increasing accuracy of the measurement or estimation of deformation of the terminal section 20, up to the distal end 30.

The extension members 34a-34c may have lengths which are equal among each other, or may have different lengths among each other.

In another embodiment in which multiple extension members are provided and coupled to the distal ending portion 32 of the optical fiber 12, the multiple extension members may be coupled to the multiple cores of the optical fiber 12. Fig. 6 shows such an embodiment, where extension members 34a, 34b, 34c (also numbered as 1, 2, 3) are connected to cores 70, 72, 74, which are outer cores of the optical fiber 12. That is, each extension member 34a, 34b, 34c will affect only one of the fiber cores 70, 72, 74 of the optical fiber 12. If the extension members 34a, 34b, 34c differ in length among each other as shown, the spatial information of the bending deformation of the terminal section 20 is transferred to the optical fiber 12 by the number of fiber cores that are strained. If the bending of the terminal section 20 is farther away from the ending portion 32 of the optical fiber 12, less fiber cores of the fiber cores 70, 72, 74 will be strained (illustrated with ' 1+2+3' for a bending closest to the ending portion 32, '2+3' for a bending midway from the ending portion 32, and '3' for a bending farthest away from the ending portion 32). The locations 54, 56, 58 of the termination of the extension members 34a, 34b, 34c of the embodiment of Fig. 5 may also be provided in the embodiment of Fig. 6.

A spatial resolution of the shape measurement or estimation of the shape of the terminal section 20 may also be achieved by a single extension member which has different strain responses to bending along a length of the extension member. Such an embodiment is shown in Fig. 7. In Fig. 7, the shell 16 of the elongated device 10 has been omitted for the sake of simplicity.

In the embodiment of Fig. 7, the mechanical extension member 34 has a varying cross-section shape, a varying cross-section diameter and a varying symmetry/asymmetry cross-section along the length of the extension member 34. Thereby, the extension member 34 has sections or segments along its length which respond differently to bending and will exert different types of strain on the optical fiber 12. For example, an extension member 34, which may be made as one monolithic part or assembled from separate parts, with three different cross-sections may be connected to the distal ending portion 32 of the optical fiber 12. The extension member 34 can induce bending strain, extension/compression strain and twist strain into the optical fiber 12 through different cross-sectional shapes. For example, close to the distal end of the ending portion 32 of the optical fiber 12, the extension member 34 may have a segment 80 having a relatively large diameter which will induce bending strain in the ending portion 32 of the optical fiber 12. Further distally, in a segment 82 of the extension member 34, the diameter of the extension member 34 may be reduced and located eccentrically with respect to the neutral bending plane, which is the bending plane coaxial with the longitudinal center axis of the optical fiber 12, such that only extension/compression is transferred to the distal ending portion 32 of the optical fiber. In a further segment 84 which is the most distal segment of the extension member 34, the extension member 34 may have an asymmetric cross-section with an eccentric shear center such that bending of the extension member 34 in that segment causes a twisting strain, i.e. a bend-twist coupling as described above, that is propagated to the optical fiber 12 through the symmetric cross-section segments 82, 80 of the extension member 34 connected to the ending portion 32 of the optical fiber 12.

Again, the bending direction of the extension member 34 may be aligned with the thin axis of a ribbon 50 as described above with respect to the embodiment of Fig. 3.

The combined strains at the ending portion 32 of the optical fiber, i.e. bending strain, axial strain and/or twist strain, can be related to both magnitude, direction and location of the bending deformation in the terminal section 20 of the elongated device 20 distally from the ending portion 32 of the optical fiber 12.

It holds for all embodiments described above that although this mechanical coupling by means of an extension member 34 or a multitude of extension members 34a, 34b, 34c is not as accurate as direct strain sensing with the optical fiber 12, the distal terminal section 20 can easily prolapse since the extension member(s) is/are much more robust than any glass fiber.

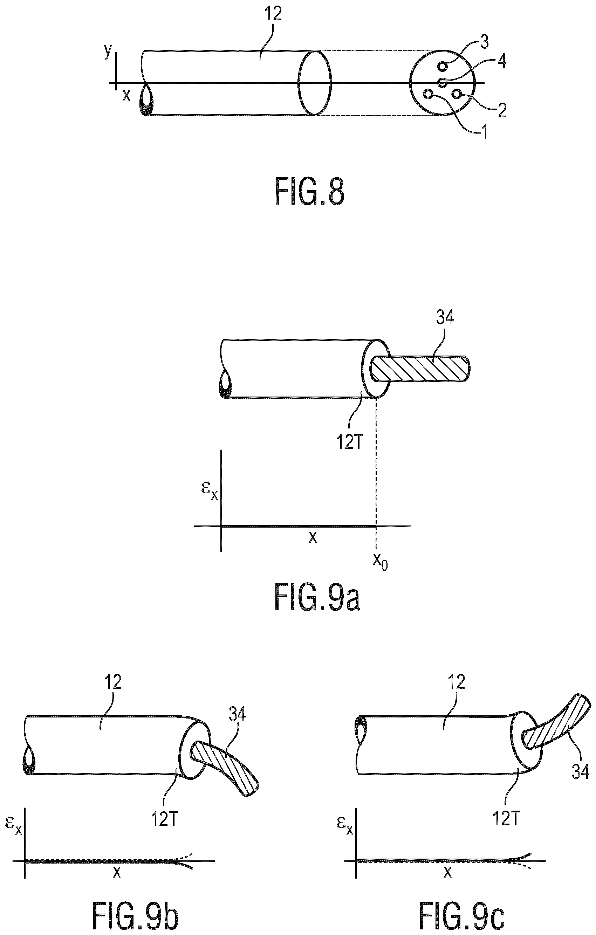

With reference to Figs. 8 to 10, an example of an extrapolation algorithm, by which a shape of an extension member 34 and, thus of the terminal section 20 of the device 10, can be determined from bending information sensed by the optical fiber 12, will be described.

Fig. 8 shows schematically a multicore optical fiber 12 having fiber cores 1, 2, 3, 4. Fiber cores 1,2,3 are outer cores, and fiber core 4 is a central core. Fig. 9 a), b) c) show the optical fiber 12 with an extension member 34 coupled to the optical 12 as described above.

Shape extrapolation can be determined by calculating the bending (and optionally axial, torsional) loading from the 4 sensing cores 1,2,3,4 up to and at the tip 12T of the optical fiber 12. The difference in loading due to the deformation of the extension member 34 will be measurable at the distal end of the optical fiber 12, but once the stiffness of the optical fiber 12 is higher than that of the extension member 34, the effect on the optical fiber shape is very limited (so the error in the shape sensing of the optical fiber 12 itself is small). Therefore, the bending stiffness (flexural rigidity) of the extension member 34 must be chosen such that it is high enough to induce some measurable strain in the tip 12T of the optical fiber 12 but it will not influence the shape of the whole optical fiber 12. This is shown schematically in Figs. 9a), 9b) and 9c), wherein the diagrams show the strain $\varepsilon_x$ in the optical fiber 12 along the longitudinal axis x. xo is the position of the coupling of the extension member 34 to the fiber 12 (note the strain $\varepsilon_x$ of a multicore fiber *without twist* is shown in Fig. 9a), b), c) for simplicity).

Normally, the bending curvature in an element, $\rho$ (= 1/R), is equal to EI/M, where EI is the flexural rigidity of the element and M is the internal bending moment in the element.

For a multicore optical fiber, the curvature, $\rho$, and direction of the axis (in physical space) along which the optical fiber is deforming can be calculated. The direction of this axis is assumed to be constant along the extension member 34 (the x-axis of the extension member), i.e. extending further in the same direction as the distal end of the optical fiber 12 (the furthest distal part that can be shape sensed). The magnitude of $\rho$ is calculated from the bending strain (computed from the measured strains $\varepsilon_x$ in the outer cores 1,2,3), and the flexural rigidity of the sensor is known (determined by design).

For an optical fiber 12 with an extension member 34 coupled thereto, the difference in measured strain and expected strain in the optical fiber 12 without an extension member 34 is a measure for the deformation of the extension member.

The curvature of an optical fiber 12 without an extension member 34 will always be zero at the distal end because the bending moment M goes to zero at the distal end (free clamping condition).

For an optical fiber 12 with an extension member 34, the distal end 12T of the optical fiber 12 can be subjected to a moment (exerted by the extension member 34) which can be measured at the distal end 12T of the optical fiber 12 (i.e. at xo) as a nonzero bending strain, $\varepsilon_{x,x0}$. Note the notation $\varepsilon_{x,x0}$ means the bending strain (measured at the radial location of the outer cores 1,2,3) at the position xo.

This moment (computed from the bending strains in the sensor cores 1,2,3) can be used to approximate the bending deformation of the extension member 34 (averaged over its length).

An example of an algorithm for approximating the shape of the device tip will be given in general terms for an extension member 34 as in the embodiment of Fig. 1:

IF $\varepsilon_{x,x0} \neq 0$, $y_{ext}(x) = f(\varepsilon_{x,x0}, x)$, for a given geometry of the extension member 34 and design of the shell 16. $y(x)$ is the deflection in the plane perpendicular to the principal bending direction at the distal end 12T of the optical fiber 12, from x = 0 (the proximal end of the extension member 34) and x = $L_{ext}$ (the distal end of the extension member 34).

The shape of the tip of the elongated device 10 can be further approximated for a known deformation and device geometry, which determines the actual shape of the device distal tip, such that: $y_{tip}(x) = f(y_{ext}(x), x)$, from x = 0 (the proximal end of the extension member 34) and x = $L_{tip}$ (the distal end 30 of the device 10), i.e. further extrapolating the calculated shape of the extension member beyond x = $L_{ext}$ (see Fig. 1, where the distal end 30 is spaced apart from the distal end of the extension member 34).

Note for other embodiments, the axial strain (i.e. from the inner core 4) and the twist (computed from the 3 outer cores) can also be used as input for $y_{ext}(x)$ in addition to the bending strain.

Furthermore, to improve the accuracy, the region in which the strain is measured in the optical fiber 12 may be larger than only the distal position (xo- D, $x_0$) and an algorithm may be applied to more accurately calculate the strain at xo.

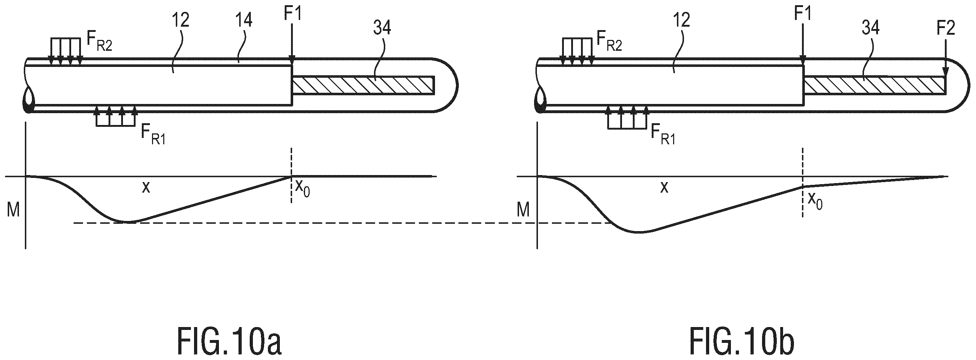

As a typical example, the case for an optical fiber 12 inside an inner lumen 14 of the elongated device 10, in particular which may be a guidewire, with a point load (F1) at the distal end is illustrated in Fig. 10a) and b). More proximally, the optical fiber experiences (distributed) loading condition ($F_{R1}$, $F_{R2}$), when the optical fiber 12 touches the inner lumen of the shell 16 giving it a certain shape (this is not important for the algorithm). From this shape calculation the vector of the centerline of the optical fiber 12 at its distal end (xo) is known.

In Fig. 10a) and b) the load cases are shown. For a straight, undeformed device tip, there is no load on the extension member 34, i.e. similar to the optical fiber 12 without an extension member and the moment M (and thus bending strain) will always be zero at xo (the most distal FBG location in the sensor). As an example for a deformed device tip, a load case where a single end load (F2) is acting on the optical fiber 12 via the extension member 34 is shown in Fig. 10b) as an example. Then, the (extrapolated) bending deflection of the extension member 34 (with respect to the distal end of the optical fiber 12) is determined by the following algorithm:

IF $\varepsilon_{x,x0} \neq 0$, $y_{ext}(x) = \dfrac{\varepsilon_{x,x0}\,(EI)_{sensor}\,x^2\,(3L_{ext}-x)}{6\,r\,(EI)_{ext}\,L_{ext}}$, where $L_{ext}$ is the length of the extension member. $\varepsilon_{x,x0}$ is the bending strain in the optical fiber 12 at its distal end, $r$ is the radial distance of the strain measurement position (i.e. the outer core position) in the optical fiber 12 and $y_{ext}$ is the radial deflection from centerline in the principal plane of bending (i.e. the tangential shape vector from the distal end of the optical fiber 12). $(EI)_{sensor}$ is the flexural rigidity of the optical fiber 12 and $(EI)_{ext}$ is the flexural rigidity of the extension member 34. The shape, calculated from x and $y_{ext}(x)$ can now be stitched onto the shape of the optical fiber 12 to show the shape of the whole device 10 beyond the distal end of the optical fiber 12.

From the previous description follows that the value of $(EI)_{ext}$ must be chosen correctly for the range and resolution of $y_{ext}$ that is desired.

The upper and lower limits of $(EI)_{ext}$ are determined by considering the measurable strain resolution of $\varepsilon_x$ in the optical fiber 12 (the lower limit of $(EI)_{ext}$) and either the bending stiffness of the device tip or the maximum error induced in the shape sensing at the distal part of the optical fiber 12 (the upper limit of $(EI)_{ext}$).

As an example, $(EI)_{ext}$ may be about 1/10 to 1/2 of $(EI)_{sensor}$ but values below this range are possible depending on the sensitivity of the optical fiber 12.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

**Claims**

1. Elongated device for medical interventional application, comprising:

   a main section (18) and a distal terminal section (20) having a bending stiffness lower than that of the main section (18),
   an optical fiber (12) arranged for sensing shape and arranged in the main section (18),
   a mechanically bendable extension member (34) coupled to a distal ending portion (32) of the optical fiber (12), the extension member (34) longitudinally extending within the terminal section (20), the extension member (34) being configured to transfer bending information related to a bending of the extension member (34) to the ending portion (32) of the optical fiber (12) such that the bending information is sensed by the optical fiber (12).

2. Elongated device according to claim 1, wherein the extension member (34) extends from the ending portion (32) of the optical fiber (12) up to a distal end (30) of the terminal section (20).

3. Elongated device according to claim 1 or 2, wherein the extension member (34) is a stainless steel or a Nitinol elongated element.

4. Elongated device according to any one of claims 1 to 3, wherein an inner wall of at least one of the main section (18) and the terminal section (20) has one or more protrusions (42, 44) at pre-determined positions along the at least one of the main section (18) and terminal section (20) to limit movement of at least one of the extension member (34) and the optical fiber (12) in one or more directions transverse to a longitudinal axis of the extension member (34) or optical fiber (12).

5. Elongated device according to any one of claims 1 to 4, wherein a distal end (30) of the terminal section (20) is configured to restrict movement of the distal end of the extension member (34) in one or more directions transverse to a longitudinal axis of the extension member (34).

6. Elongated device according to any one of claims 1 to 5, wherein the extension member (34) is coupled to the optical fiber (12) eccentrically with respect to a longitudinal center axis of the optical fiber (12).

7. Elongated device according to any one of claims 1 to 6, wherein a distal end of the extension member (34) is connected to a distal end (30) of the terminal section (20) eccentrically with respect to a longitudinal center axis of the lumen (14).

8. Elongated device according to any one of claims 1 to 7, further comprising a ribbon (50) arranged along an inner wall of the terminal section (20)and having a first bending resistance in two opposite first bending directions (46, 48) and a second bending resistance in two opposite second bending directions transverse to the first bending directions (46, 48), the first bending resistance being smaller than the second bending resistance, and wherein the extension member (34) is arranged with respect to the ribbon (50) such that the first bending directions are the preferential bending directions of the terminal section (20)

9. Elongated device according to any one of claims 1 to 8, wherein the extension member (34) and/or a coupling element arranged between the distal ending portion (32) of the optical fiber (12) and the extension member (34) has an asymmetric cross-section such that bending of the extension member (34) or coupling element induces twist in the ending portion (32) of the optical fiber (12) which twist is sensed by the optical fiber (12).

10. Elongated device according to any one of claims 1 to 9, comprising a plurality of extension members (34a, 34b, 34c), the extension members (34a, 34b, 34c) being spatially distributed and coupled to the ending portion (32) of the optical fiber (12).

11. Elongated device according to claim 10, wherein the extension members (34a, 34b, 34c) are coupled to the ending portion (32) of the optical fiber (12) at different axial and/or circumferential positions (60, 62, 64) along or around the ending portion (32) of the optical fiber (12).

**12.** Elongated device according to claim 10 or 11, wherein the extension members (34a, 34b, 34c) of the plurality of extension members terminate at different axial and/or circumferential positions (54, 56, 58) along or around a longitudinal axis of the terminal section (20).

**13.** Elongated device according to any one of claims 10 to 12, wherein the extension members (34a, 34b, 34c) are coupled to different cores (70, 72, 74) of the ending portion (32) of the optical fiber (12).

**14.** Elongated device according to any one of claims 10 to 13, wherein the extension members (34a, 34b, 34c) have different lengths among each other.

**15.** Elongated device according to any one of claims 1 to 14, wherein the extension member (34) is configured to provide different strain responses to bending along a length of the extension member (34), wherein the extension member (34) comprises at least one of a varying cross-section diameter along the length of the extension member (34), a varying cross-section shape along the length of the extension member (34), a varying cross-section symmetry or asymmetry along the length of the extension member (34).

FIG.1

FIG.2

FIG.3

34

a)

34

b)

34

e

c)

34

θ

θ

e

r

d)

FIG.4

FIG.5

FIG.6

FIG.7

FIG.8

FIG.9a

FIG.9b

FIG.9c

FIG.10a

FIG.10b

## EUROPEAN SEARCH REPORT

**Application Number**

EP 24 20 7530

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/211112 A1 (KONINKLIJKE PHILIPS NV [NL]) 7 November 2019 (2019-11-07) | 1-5,8, 10,11, 13,15 | INV.<br>A61B1/005<br>A61B34/20 |
| Y | * paragraphs [0001], [0042] - [0044], [0060]; claim 1; figures 4,13 * | 6,7,9, 12,14 | G01B11/24 |
| Y | WO 2013/116140 A1 (INTUITIVE SURGICAL OPERATIONS [US]) 8 August 2013 (2013-08-08) | 6,7,9, 12,14 | |
| A | * paragraph [0014]; figure 3 * | 3 | |
| A | US 2016/166341 A1 (IORDACHITA IULIAN [US] ET AL) 16 June 2016 (2016-06-16) * paragraphs [0082], [0089]; figures 3,5,14 * | 3,8,10, 11,13 | |
| A | WO 2020/131576 A1 (MAKO SURGICAL CORP [US]) 25 June 2020 (2020-06-25) * claim 1 * | 1-15 | |
| A | US 2024/108210 A1 (HAMM MARK ALAN [US]) 4 April 2024 (2024-04-04) * figure 14 * | 1-15 | **TECHNICAL FIELDS SEARCHED (IPC)**<br><br>A61B<br>G01B |

The present search report has been drawn up for all claims

| Place of search | Date of completion of the search | Examiner |
|---|---|---|
| The Hague | 5 December 2024 | Chacon Caldera, J |

## ANNEX TO THE EUROPEAN SEARCH REPORT
## ON EUROPEAN PATENT APPLICATION NO.

EP 24 20 7530

This annex lists the patent family members relating to the patent documents cited in the above-mentioned European search report.
The members are as contained in the European Patent Office EDP file on
The European Patent Office is in no way liable for these particulars which are merely given for the purpose of information.

05-12-2024

| Patent document cited in search report | Publication date | Patent family member(s) | | Publication date |
|---|---|---|---|---|
| WO 2019211112 A1 | 07-11-2019 | US | 2021113274 A1 | 22-04-2021 |
| | | WO | 2019211112 A1 | 07-11-2019 |
| WO 2013116140 A1 | 08-08-2013 | EP | 2809249 A1 | 10-12-2014 |
| | | EP | 3488803 A1 | 29-05-2019 |
| | | JP | 6290099 B2 | 07-03-2018 |
| | | JP | 6847803 B2 | 24-03-2021 |
| | | JP | 2015505507 A | 23-02-2015 |
| | | JP | 2017225866 A | 28-12-2017 |
| | | US | 2013204124 A1 | 08-08-2013 |
| | | US | 2017014194 A1 | 19-01-2017 |
| | | US | 2020289023 A1 | 17-09-2020 |
| | | US | 2023148897 A1 | 18-05-2023 |
| | | WO | 2013116140 A1 | 08-08-2013 |
| US 2016166341 A1 | 16-06-2016 | NONE | | |
| WO 2020131576 A1 | 25-06-2020 | US | 2020188036 A1 | 18-06-2020 |
| | | US | 2023000569 A1 | 05-01-2023 |
| | | WO | 2020131576 A1 | 25-06-2020 |
| US 2024108210 A1 | 04-04-2024 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WANG, JIAN-BO et al.** An Effective Guidewire Looping Technique for the Recanalization of Occlusive Segments of Infrapopliteal Vessels. *Korean Journal of Radiology*, 2010, vol. 11, 441-448